# EUROPÄISCHE PATENTANMELDUNG

(11) **EP 0 902 008 A2**
(43) Veröffentlichungstag der Anmeldung: **17.03.1999**
(21) Anmeldenummer: 98116082.3
(22) Anmeldetag: 26.08.1998
(51) Int. Cl.: C07C 219/06, C07C 219/08, C07C 217/08, A61K 7/00

(54) **Wassermischbare Kationtensidkonzentrate**

(30) Priorität: 04.09.1997 DE 19738645
(71) Anmelder: Henkel Kommanditgesellschaft auf Aktien, 40589 Düsseldorf-Holthausen (DE)
(72) Erfinder: Prat, Esther, Dr., 08238 Alella (ES); Pi Subirana, Rafael, Dr., 08400 Granollers (ES); Bigorra, Joaquin Dr., 08203 Sabadell (ES)

(57) **Zusammenfassung**

Vorgeschlagen werden wassermischbare Kationtensidkonzentrate, enthaltend
(a) 75 bis 95 Gew.-% Esterquats und
(b) 5 bis 25 Gew.-% Ethanol, Ethylenglycol und/oder Propylenglycol.

Die Mischungen sind niedrigviskos und klar und lassen sich in praktisch beliebigen Verhältnissen mit Wasser mischen, wobei ebenfalls wieder trübungsfreie Lösungen erhalten werden, welche unmittelbar als kosmetische Mittel, z.B. als Haarkuren eingesetzt werden können.

## Beschreibung

### Gebiet der Erfindung

Die Erfindung betrifft wassermischbare Kationtensidkonzentrate mit definierten Gehalten an Esterquats und ausgewählten niedermolekularen Alkoholen, ein Verfahren zu deren Herstellung, kosmetische Zubereitungen, die auf Basis der Konzentrate erhalten werden sowie die Verwendung der Konzentrate zur Herstellung von kosmetischen Mitteln,

### Stand der Technik

Kationische Tenside vom Typ der Esterquats werden in der Kosmetik beispielsweise für die Haaravivage eingesetzt. Wegen ihrer guten sensorischen Eigenschaften finden diese Stoffe auch zunehmend Eingang in die Hautkosmetik. Üblicherweise gelangen die Esterquats herstellungsbedingt als konzentrierte Lösungen in Isopropylalkohol in den Handel. Für eine kosmetische Anwendung ist Isopropylalkohol nicht zugelassen, so daß die Konzentrate zunächst vom Lösungsmittel befreit werden müssen, was mit erheblichem technischen Aufwand verbunden ist. Aus der Deutschen Patentschrift **DE-C1 4308794** (Henkel) ist in diesem Zusammenhang ein Verfahren zur Herstellung fester Esterquats bekannt, bei dem man die Quaternierung von Triethanolaminestern in Gegenwart von geeigneten Dispergatoren, vorzugsweise Fettalkoholen, durchführt. In der **DE-C1 4335782** (Henkel) wird ferner vorgeschlagen, die Quaternierung von Triethanolaminfettsäureestern in Gegenwart von Polyolen, beispielsweise Glycerin, Ethylenglycol, Partialglyceriden und dergleichen durchzuführen, um die Verwendung von Isopropylalkohol als brennbarem Lösemittel zu vermeiden. Es werden dabei Produkte erhalten, die mehr oder minder flüssig, jedoch in Wasser nur unter starker Austrübung löslich sind. So führt die Quaternierung von Ditalgfettsäuretriethanolaminester mit Dimethylsulfat in Gegenwart von Glycerin zu einem viskosen Sirup, der auch noch in 5 Gew.-%iger Verdünnung mit Wasser nur eine trübe Lösung ergibt. Solche Zubereitungen lassen sich nicht zu klaren Endprodukten formulieren, was deren Einsatzmöglichkeiten in der Kosmetik stark einschränkt.

Die komplexe Aufgabe der Erfindung hat somit darin bestanden, Konzentrate von Esterquats in einem für kosmetische Zwecke geeigneten Lösemittel zur Verfügung zu stellen, die bei Raumtemperatur niedrigviskos und trübungsfrei sind und sich problemlos mit Wasser verdünnen lassen, ohne daß es dabei zu Austrübungen kommt.

### Beschreibung der Erfindung

Gegenstand der Erfindung sind wassermischbare Kationtensidkonzentrate, enthaltend
(a) 75 bis 95, vorzugsweise 80 bis 90 Gew.-% Esterquats und
(b) 5 bis 25, vorzugsweise 10 bis 20 Gew.-% Ethanol, Ethylenglycol und/oder Propylenglycol,
mit der Maßgabe, daß sich die Mengenangaben zu 100 Gew.-% ergänzen.

Überraschenderweise wurde gefunden, daß die erfindungsgemäßen Konzentrate nicht nur selbst niedrigviskos und klar sind, sondern auch in beliebiger Verdünnung mit Wasser klare Lösungen ergeben. Die aus den Konzentraten erhältlichen wäßrigen Zubereitungen lassen sich dabei unmittelbar als kosmetische Mittel beispielsweise als Haarkuren oder als Vorprodukte für Haarshampoos verwenden.

### Esterquats

Unter der Bezeichnung 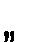 Esterquats" werden im allgemeinen quaternierte Fettsäuretriethanolaminestersalze verstanden. Es handelt sich dabei um bekannte Stoffe, die man nach den einschlägigen Methoden der präparativen organischen Chemie erhalten kann. In diesem Zusammenhang sei auf die Internationale Patentanmeldung **WO 91/01295** (Henkel) verwiesen, nach der man Triethanolamin in Gegenwart von unterphosphoriger Säure mit Fettsäuren partiell verestert, Luft durchleitet und anschließend mit Dimethylsulfat oder Ethylenoxid quaterniert. Übersichten zu diesem Thema sind beispielsweise von R.Puchta et al. in **Tens.Surf.Det., 30, 186 (1993),** M.Brock in **Tens.Surf.Det. 30**, **394 (1993)**, R.Lagerman et al. in **J.Am.Oil.Chem.Soc., 71, 97 (1994)** sowie I.Shapiro in **Cosm.Toil. 109, 77 (1994)** erschienen.

Die quaternierten Fettsäuretriethanolaminestersalze folgen der Formel **(I)**, 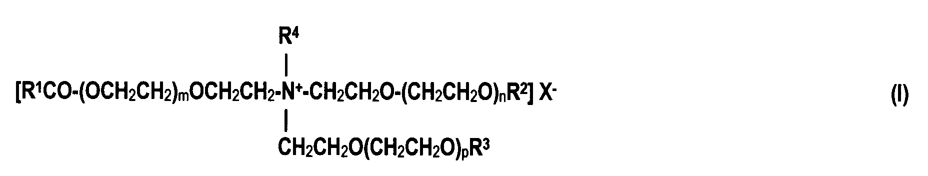 in der R¹CO für einen Acylrest mit 6 bis 22 Kohlenstoffatomen, R² und R³ unabhängig voneinander für Wasserstoff oder R¹CO, R⁴ für einen Alkylrest mit 1 bis 4 Kohlenstoffatomen oder eine (CH₂CH₂O)_{q}H-Gruppe, m, n und p in Summe für 0 oder Zahlen von 1 bis 12, q für Zahlen von 1 bis 12 und X für Halogenid, Alkylsulfat oder Alkylphosphat steht. Typische Beispiele für Esterquats, die im Sinne der Erfindung Verwendung finden können, sind Produkte auf Basis von Capronsäure, Caprylsäure, Caprinsäure, Laurinsäure, Myristinsäure, Palmitinsäure, Isostearinsäure, Stearinsäure, Ölsäure, Elaidinsäure, Arachinsäure, Behensäure und Erucasäure sowie deren technische Mischungen, wie sie beispielsweise bei der Druckspaltung natürlicher Fette und Öle anfallen. Vorzugsweise werden technische C_{12/18}-Kokosfettsäuren und insbesondere teilgehärtete C_{16/18}-Talg- bzw. Palmfettsäuren sowie elaidinsäurereiche C_{16/18}-Fettsäureschnitte eingesetzt. Zur Herstellung der quaternierten Ester können die Fettsäuren und das Triethanolamin im molaren Verhältnis von 1,1 : 1 bis 3 : 1 eingesetzt werden. Im Hinblick auf die anwendungstechnischen Eigenschaften der Esterquats hat sich ein Einsatzverhältnis von 1,2 : 1 bis 2,2 : 1, vorzugsweise 1,5 : 1 bis 1,9 : 1 als besonders vorteilhaft erwiesen. Die bevorzugten Esterquats stellen technische Mischungen von Mono-, Di- und Triestern mit einem durchschnittlichen Veresterungsgrad von 1,5 bis 1,9 dar und leiten sich von technischer C_{12/18}-Kokosfettsäure (Iodzahl 0 bis 40) ab. Aus anwendungstechnischer Sicht haben sich quaternierte Fettsäuretriethanolaminestersalze der Formel **(I)** als besonders vorteilhaft erwiesen, in der R¹CO für einen Acylrest mit 12 bis 18 Kohlenstoffatomen, R² für R¹CO, R³ für Wasserstoff, R⁴ für eine Methylgruppe, m, n und p für 0 und X für Methylsulfat steht.

Neben den quaternierten Fettsäuretriethanolaminestersalzen kommen als Esterquats ferner auch quaternierte Estersalze von Fettsäuren mit Diethanolalkylaminen der Formel **(II)** in Betracht, 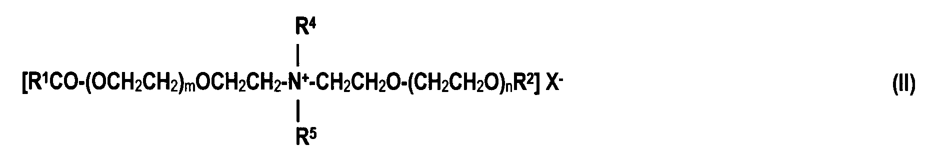 in der R¹CO für einen Acylrest mit 6 bis 22 Kohlenstoffatomen, R² für Wasserstoff oder R¹CO, R⁴ und R⁵ unabhängig voneinander für Alkylreste mit 1 bis 4 Kohlenstoffatomen, m und n in Summe für 0 oder Zahlen von 1 bis 12 und X für Halogenid, Alkylsulfat oder Alkylphosphat steht.

Als weitere Gruppe geeigneter Esterquats sind schließlich die quaternierten Estersalze von Fettsäuren mit 1,2-Dihydroxypropyldialkylaminen der Formel **(III)** zu nennen, 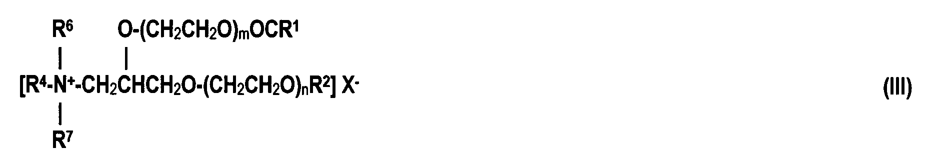 in der R¹CO für einen Acylrest mit 6 bis 22 Kohlenstoffatomen, R² für Wasserstoff oder R¹CO, R⁴, R⁶ und R⁷ unabhängig voneinander für Alkylreste mit 1 bis 4 Kohlenstoffatomen, m und n in Summe für 0 oder Zahlen von 1 bis 12 und X für Halogenid, Alkylsulfat oder Alkylphosphat steht.

Hinsichtlich der Auswahl der bevorzugten Fettsäuren und des optimalen Veresterungsgrades gelten die für **(I)** genannten Beispiele auch für die Esterquats der Formeln **(II)** und **(III)**.

### Herstellverfahren

Ein weiterer Gegenstand der Erfindung betrifft ein Verfahren zur Herstellung der genannten Konzentrate, bei dem man Alkanolaminfettsäureester in Gegenwart von solchen Mengen Ethanol, Ethylenglycol, Propylenglycol oder deren Gemischen mit Alkylierungsmitteln umsetzt, daß sich ein Gewichtsverhältnis Esterquat : Lösemittel von 95 : 5 bis 75 : 25 und vorzugsweise 90 : 10 bis 80 : 20 ergibt. Aus anwendungstechnischer Sicht ist der Einsatz von Ethanol sowie von Gemischen aus Ethanol und Propylenglycol im Gewichtsverhältnis 10 : 90 bis 90 : 10 und insbesondere 75 : 25 bis 25 : 75 bevorzugt. Die Veresterung und Quaternierung kann dabei in an sich bekannter Weise durchgeführt werden, wie dies beispielsweise ausführlich in den bereits eingangs genannten Druckschriften DE-C1 4308794 und DE-C1 4335782 beschrieben wird.

Ein weiterer Gegenstand betrifft kosmetische Zubereitungen, enthaltend
(a) 40 bis 75 Gew.-% der beanspruchten Konzentrate und
(b) 25 bis 60 Gew.-% Wasser.
mit der Maßgabe, daß sich die Mengenangaben zu 100 Gew.-% ergänzen.

Die Zubereitungen werden durch einfaches Verdünnen mit Wasser hergestellt und zeichnen sich vor allem dadurch aus, daß sie klar, niedrigviskos und lagerstabil sind, d.h. daß sie während mehrwöchentlicher Lagerung weder eindicken noch austrüben. Der Wasseranteil in diesen Zubereitungen liegt vorzugsweise bei 30 bis 50 und insbesondere 40 bis 45 Gew.-%. Die Lösungen können beispielsweise unmittelbar als Haarkuren eingesetzt werden, sie dienen aber auch als Vorprodukte beispielsweise für die Herstellung von Haarshampoos.

### Gewerbliche Anwendbarkeit

Ein letzter Gegenstand der Erfindung betrifft die Verwendung der genannten Konzentrate nach zur Herstellung von kosmetischen Zubereitungen. Im einfachsten Fall können die Konzentrate als solche bzw. in wäßriger Verdünnung eingesetzt werden. Sie können jedoch auch je nach Anwendungszweck zusammen mit weiteren für kosmetische Anwendungen typischen Inhaltsstoffen formuliert werden, wie beispielsweise milde Tenside, Ölkörper, Emulgatoren, Überfettungsmittel, Perlglanzwachse, Stabilisatoren, Konsistenzgeber, Verdickungsmittel, Kationpolymere, Siliconverbindungen, biogene Wirkstoffe, Antischuppenmittel, Filmbildner, Konservierungsmittel, Hydrotrope, Solubilisatoren, UV-Lichtschutzfilter, Insektenrepellentien, Selbstbräuner, Parfümöle, Farbstoffe und dergleichen.

Typische Beispiele für geeignete milde, d.h. besonders hautverträgliche **Tenside** sind Fettalkoholpolyglycolethersulfate, Monoglyceridsulfate, Mono- und/oder Dialkylsulfosuccinate, Fettsäureisethionate, Fettsäuresarcosinate, Fettsäuretauride, Fettsäureglutamate, Ethercarbonsäuren, Alkyloligoglucoside, Fettsäureglucamide, Alkylamidobetaine und/oder Proteinfettsäurekondensate, letztere vorzugsweise auf Basis von Weizenproteinen.

Als **Ölkörper** kommen beispielsweise Guerbetalkohole auf Basis von Fettalkoholen mit 6 bis 18, vorzugsweise 8 bis 10 Kohlenstoffatomen, Ester von linearen C₆-C₂₂-Fettsäuren mit linearen C₆-C₂₂-Fettalkoholen, Ester von verzweigten C₆-C₁₃-Carbonsäuren mit linearen C₆-C₂₂-Fettalkoholen, Ester von linearen C₆-C₂₂-Fettsäuren mit verzweigten Alkoholen, insbesondere 2-Ethylhexanol, Ester von linearen und/oder verzweigten Fettsäuren mit mehrwertigen Alkoholen (wie z.B. Propylenglycol, Dimerdiol oder Trimertriol) und/oder Guerbetalkoholen, Triglyceride auf Basis C₆-C₁₀-Fettsäuren, flüssige Mono-/Di-Triglyceridmischungen auf Basis von C₆-C₁₈-Fettsäuren, Ester von C₆-C₂₂-Fettalkoholen und/oder Guerbetalkoholen mit aromatischen Carbonsäuren, insbesondere Benzoesäure, pflanzliche Öle, verzweigte primäre Alkohole, substituierte Cyclohexane, lineare C₆-C₂₂-Fettalkoholcarbonate, Guerbetcarbonate, Ester der Benzoesäure mit linearen und/oder verzweigten C₆-C₂₂-Alkoholen (z.B. Finsolvo® TN), Dialkylether, Ringöffnungsprodukte von epoxidierten Fettsäureestern mit Polyolen, Siliconöle und/oder aliphatische bzw. naphthenische Kohlenwasserstoffe in Betracht.

Als **Emulgatoren** kommen beispielsweise nichtionogene Tenside aus mindestens einer der folgenden Gruppen in Frage:
(1) Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe;
(2) C_{12/18}-Fettsäuremono- und -diester von Anlagerungsprodukten von 1 bis 30 Mol Ethylenoxid an Glycerin;
(3) Glycerinmono- und -diester und Sorbitanmono- und -diester von gesättigten und ungesättigten Fettsäuren mit 6 bis 22 Kohlenstoffatomen und deren Ethylenoxidanlagerungsprodukte;
(4) Alkylmono- und -oligoglycoside mit 8 bis 22 Kohlenstoffatomen im Alkylrest und deren ethoxylierte Analoga;
(5) Anlagerungsprodukte von 15 bis 60 Mol Ethylenoxid an Ricinusöl und/oder gehärtetes Ricinusöl;
(6) Polyol- und insbesondere Polyglycerinester wie z.B. Polyglycerinpolyricinoleat oder Polyglycerinpoly-12-hydroxystearat. Ebenfalls geeignet sind Gemische von Verbindungen aus mehreren dieser Substanzklassen;
(7) Anlagerungsprodukte von 2 bis 15 Mol Ethylenoxid an Ricinusöl und/oder gehärtetes Ricinusöl;
(8) Partialester auf Basis linearer, verzweigter, ungesättigter bzw. gesättigter C_{6/22}-Fettsäuren, Ricinolsäure sowie 12-Hydroxystearinsäure und Glycerin, Polyglycerin, Pentaerythrit, Dipentaerythrit, Zuckeralkohole (z.B. Sorbit), Alkylglucoside (z.B. Methylglucosid, Butylglucosid, Laurylglucosid) sowie Polyglucoside (z.B. Cellulose);
(9) Trialkylphosphate sowie Mono-, Di- und/oder Tri-PEG-alkylphosphate;
(10) Wollwachsalkohole;
(11) Polysiloxan-Polyalkyl-Polyether-Copolymere bzw. entsprechende Derivate;
(12) Mischester aus Pentaerythrit, Fettsäuren, Citronensäure und Fettalkohol gemäß **DE-PS 1165574** und/oder Mischester von Fettsäuren mit 6 bis 22 Kohlenstoffatomen, Methylglucose und Polyolen, vorzugsweise Glycerin sowie
(13) Polyalkylenglycole.

Die Anlagerungsprodukte von Ethylenoxid und/oder von Propylenoxid an Fettalkohole, Fettsäuren, Alkylphenole, Glycerinmono- und -diester sowie Sorbitanmono- und -diester von Fettsäuren oder an Ricinusöl stellen bekannte, im Handel erhältliche Produkte dar. Es handelt sich dabei um Homologengemische, deren mittlerer Alkoxylierungsgrad dem Verhältnis der Stoffmengen von Ethylenoxid und/oder Propylenoxid und Substrat, mit denen die Anlagerungsreaktion durchgeführt wird, entspricht. C_{12/18}-Fettsäuremono- und -diester von Anlagerungsprodukten von Ethylenoxid an Glycerin sind aus **DE-PS 20 24 051** als Rückfettungsmittel für kosmetische Zubereitungen bekannt.

C_{8/18}-Alkylmono- und -oligoglycoside, ihre Herstellung und ihre Verwendung als oberflächenaktive Stoffe sind literaturbekannt. Ihre Herstellung erfolgt insbesondere durch Umsetzung von Glucose oder Oligosacchariden mit primären Alkoholen mit 8 bis 18 C-Atomen. Bezüglich des Glycosidrestes gilt, daß sowohl Monoglycoside, bei denen ein cyclischer Zuckerrest glycosidisch an den Fettalkohol gebunden ist, als auch oligomere Glycoside mit einem Oligomerisationsgrad bis vorzugsweise etwa 8 geeignet sind. Der Oligomerisierungsgrad ist dabei ein statistischer Mittelwert, dem eine für solche technischen Produkte übliche Homologenverteilung zugrunde liegt.

Weiterhin können als Emulgatoren zwitterionische Tenside verwendet werden. Als zwitterionische Tenside werden solche oberflächenaktiven Verbindungen bezeichnet, die im Molekül mindestens eine quartäre Ammoniumgruppe und mindestens eine Carboxylat- und eine Sulfonatgruppe tragen. Besonders geeignete zwitterionische Tenside sind die sogenannten Betaine wie die N-Alkyl-N,N-dimethylammoniumglycinate, beispielsweise das Kokosalkyldimethylammoniumglycinat, N-Acylamino-propyl-N,N-dimethylammoniumglycinate, beispielsweise das Kokosacylaminopropyldimethylammonium-glycinat, und 2-Alkyl-3-carboxylmethyl-3-hydroxyethylimidazoline mit jeweils 8 bis 18 C-Atomen in der Alkyl- oder Acylgruppe sowie das Kokosacylaminoethylhydroxyethylcarboxymethylglycinat. Besonders bevorzugt ist das unter der CTFA-Bezeichnung *Cocamidopropyl* Betaine bekannte Fettsäureamid-Derivat. Ebenfalls geeignete Emulgatoren sind ampholytische Tenside. Unter ampholytischen Tensiden werden solche oberflächenaktiven Verbindungen verstanden, die außer einer C_{8/18}-Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C_{12/18}-Acylsarcosin. Neben den ampholytischen kommen auch quartäre Emulgatoren in Betracht, wobei solche vom Typ der Esterquats, vorzugsweise methylquaternierte Difettsäuretriethanolaminester-Salze, besonders bevorzugt sind.

Als **Überfettungsmittel** können Substanzen wie beispielsweise Lanolin und Lecithin sowie polyethoxylierte oder acylierte Lanolin- und Lecithinderivate, Polyolfettsäureester, Monoglyceride und Fettsäurealkanolamide verwendet werden, wobei die letzteren gleichzeitig als Schaumstabilisatoren dienen.

Als **Perlglanzwachse** kommen beispielsweise in Frage : Alkylenglycolester, speziell Ethylenglycoldistearat; Fettsäurealkanolamide, speziell Kokosfettsäurediethanolamid; Partialglyceride, speziell Stearinsäuremonoglycerid; Ester von mehrwertigen, gegebenenfalls hydroxysubstituierte Carbonsäuren mit Fettalkoholen mit 6 bis 22 Kohlenstoffatomen, speziell langkettige Ester der Weinsäure; Fettstoffe, wie beispielsweise Fettalkohole, Fettketone, Fettaldehyde, Fettether und Fettcarbonate, die in Summe mindestens 24 Kohlenstoffatome aufweisen, speziell Lauron und Distearylether; Fettsäuren wie Stearinsäure, Hydroxystearinsäure oder Behensäure, Ringöffnungsprodukte von Olefinepoxiden mit 12 bis 22 Kohlenstoffatomen mit Fettalkoholen mit 12 bis 22 Kohlenstoffatomen und/oder Polyolen mit 2 bis 15 Kohlenstoffatomen und 2 bis 10 Hydroxylgruppen; sowie deren Mischungen.

Als **Konsistenzgeber** kommen in erster Linie Fettalkohole mit 12 bis 22 und vorzugsweise 16 bis 18 Kohlenstoffatomen und daneben Partialglyceride in Betracht. Bevorzugt ist eine Kombination dieser Stoffe mit Alkyloligoglucosiden und/oder Fettsäure-N-methylglucamiden gleicher Kettenlänge und/oder Polyglycerinpoly-12-hydroxystearaten. Geeignete **Verdickungsmittel** sind beispielsweise Polysaccharide, insbesondere Xanthan-Gum, Guar-Guar, Agar-Agar, Alginate und Tylosen, Carboxymethylcellulose und Hydroxyethylcellulose, ferner höhermolekulare Polyethylenglycolmono- und -di-ester von Fettsäuren, Polyacrylate, (z.B. Carbopole® von Goodrich oder Synthalene® von Sigma), Polyacrylamide, Polyvinylalkohol und Polyvinylpyrrolidon, Tenside wie beispielsweise ethoxylierte Fettsäureglyceride, Ester von Fettsäuren mit Polyolen wie beispielsweise Pentaerythrit oder Trimethylolpropan, Fettalkoholethoxylate mit eingeengter Homologenverteilung oder Alkyloligoglucoside sowie Elektrolyte wie Kochsalz und Ammoniumchlorid.

Geeignete **kationische Polymere** sind beispielsweise kationische Cellulosederivate, wie z.B. ein quaternierte Hydroxyethylcellulose, die unter der Bezeichnung Polymer JR 400® von Amerchol erhältlich ist, kationische Stärke, Copolymere von Diallylammoniumsalzen und Acrylamiden, quaternierte Vinylpyrrolidon/Vinyl-imidazol-Polymere wie z.B. Luviquat® (BASF), Kondensationsprodukte von Polyglycolen und Aminen, quaternierte Kollagenpolypeptide wie beispielsweise Lauryldimonium hydroxypropyl hydrolyzed collagen (Lamequat®L/Grünau), quaternierte Weizenpolypeptide, Polyethylenimin, kationische Siliconpolymere wie z.B. Amidomethicone, Copolymere der Adipinsäure und Dimethylaminohydroxypropyldiethylentriamin (Cartaretine®/Sandoz), Copolymere der Acrylsäure mit Dimethyldiallylammoniumchlorid (Merquat® 550/Chemviron), Polyaminopolyamide wie z.B. beschrieben in der **FR-A 2252840** sowie deren vernetzte wasserlöslichen Polymere, kationische Chitinderivate wie beispielsweise quaterniertes Chitosan, gegebenenfalls mikrokristallin verteilt, Kondensationsprodukte aus Dihalogenalkylen wie z.B. Dibrombutan mit Bisdialkylaminen wie z.B. Bis-Dimethylamino-1,3-propan, kationischer Guar-Gum wie z.B. Jaguar® CBS, Jaguar® C-17, Jaguar® C-16 der Celanese, quaternierte Ammoniumsalz-Polymere wie z.B. Mirapol® A-15, Mirapol® AD-1, Mirapol® AZ-1 der Miranol.

Geeignete **Siliconverbindungen** sind beispielsweise Dimethylpolysiloxane, Methyl-phenylpolysiloxane, cyclische Silicone sowie amino-, fettsäure-, alkohol-, polyether-, epoxy-, fluor- und/oder alkylmodifizierte Siliconverbindungen, die bei Raumtemperatur sowohl flüssig als auch harzförmig vorliegen können. Typische Beispiele für **Fette** sind Glyceride, als **Wachse** kommen u.a. Bienenwachs, Carnaubawachs, Candelillawachs, Montanwachs, Paraffinwachs oder Mikrowachse gegebenenfalls in Kombination mit hydrophilen Wachsen, z.B. Cetylstearylalkohol oder Partialglyceriden in Frage. Als **Stabilisatoren** können Metallsalze von Fettsäuren wie z.B. Magnesium-, Aluminium- und/oder Zinkstearat eingesetzt werden. Unter **biogenen Wirkstoffen** sind beispielsweise Tocopherol, Tocopherolacetat, Tocopherolpalmitat, Ascorbinsäure, Retinol, Bisabolol, Allantoin, Phytantriol, Panthenol, AHA-Säuren, Aminosäuren, Ceramide, Pseudoceramide, essentielle Öle, Pflanzenextrakte und Vitaminkomplexe zu verstehen. Als **Antischuppenmittel** können Climbazol, Octopirox und Zinkpyrethion eingesetzt werden. Gebräuchliche **Filmbildner** sind beispielsweise Chitosan, mikrokristallines Chitosan, quaterniertes Chitosan, Polyvinylpyrrolidon, Vinyl-pyrrolidon-Vinylacetat-Copolymerisate, Polymere der Acrylsäurereihe, quaternäre Cellulose-Derivate, Kollagen, Hyaluronsäure bzw. deren Salze und ähnliche Verbindungen. Als **Quellmittel** für wäßrige Phasen können Montmorillonite, Clay Mineralstoffe, Pemulen sowie alkylmodifizierte Carbopoltypen (Goodrich) dienen.

Unter **UV-Lichtschutzfiltern** sind organische Substanzen zu verstehen, die in der Lage sind, ultraviolette Strahlen zu absorbieren und die aufgenommene Energie in Form längerwelliger Strahlung, z.B. Wärme wieder abzugeben. Typische Beispiele sind 4-Aminobenzoesäure sowie ihre Ester und Derivate (z.B. 2-Ethylhexyl-p-dimethylaminobenzoat oder p-Dimethylaminobenzoesäureoctylester), Methoxyzimtsäure und ihre Derivate (z.B. 4-Methoxyzimtsäure-2-ethylhexylester), Benzophenone (z.B. Oxybenzon, 2-Hydroxy-4-methoxybenzophenon), Dibenzoylmethane, Salicylatester, 2-Phenylbenzimidazol-5-sulfonsäure, 1-(4-tert.Butylphenyl)-3-(4'-methoxyphenyl)-propan-1,3-dion, 3-(4'-Methyl)benzylidenbornan-2-on, Methylbenzylidencampher und dergleichen. Weiterhin kommen für diesen Zweck auch feindisperse Metalloxide bzw. Salze in Frage, wie beispielsweise Titandioxid, Zinkoxid, Eisenoxid, Aluminiumoxid, Ceroxid, Zirkoniumoxid, Silicate (Talk) und Bariumsulfat. Die Partikel sollten dabei einen mittleren Durchmesser von weniger als 100 nm, vorzugsweise zwischen 5 und 50 nm und insbesondere zwischen 15 und 30 nm aufweisen. Sie können eine sphärische Form aufweisen, es können jedoch auch solche Partikel zum Einsatz kommen, die eine ellipsoide oder in sonstiger Weise von der sphärischen Gestalt abweichende Form besitzen. Neben den beiden vorgenannten Gruppen primärer Lichtschutzstoffe können auch sekundäre Lichtschutzmittel vom Typ der Antioxidantien eingesetzt werden, die die photochemische Reaktionskette unterbrechen, welche ausgelöst wird, wenn UV-Strahlung in die Haut eindringt. Typische Beispiele hierfür sind Superoxid-Dismutase, Tocopherole (Vitamin E) und Ascorbinsäure (Vitamin C).

Zur Verbesserung des Fließverhaltens können ferner **Hydrotrope** wie beispielsweise Ethanol, Isopropylalkohol, oder Polyole eingesetzt werden. Polyole, die hier in Betracht kommen, besitzen vorzugsweise 2 bis 15 Kohlenstoffatome und mindestens zwei Hydroxylgruppen. Typische Beispiele sind
- Glycerin;
- Alkylenglycole wie beispielsweise Ethylenglycol, Diethylenglycol, Propylenglycol, Butylenglycol, Hexylenglycol sowie Polyethylenglycole mit einem durchschnittlichen Molekulargewicht von 100 bis 1.000 Dalton;
- technische Oligoglyceringemische mit einem Eigenkondensationsgrad von 1,5 bis 10 wie etwa technische Diglyceringemische mit einem Diglyceringehalt von 40 bis 50 Gew.-%;
- Methyolverbindungen, wie insbesondere Trimethylolethan, Trimethylolpropan, Trimethylolbutan, Pentaerythrit und Dipentaerythrit;
- Niedrigalkylglucoside, insbesondere solche, mit 1 bis 8 Kohlenstoffen im Alkylrest wie beispielsweise Methyl- und Butylglucosid;
- Zuckeralkohole mit 5 bis 12 Kohlenstoffatomen wie beispielsweise Sorbit oder Mannit,
- Zucker mit 5 bis 12 Kohlenstoffatomen wie beispielsweise Glucose oder Saccharose;
- Aminozucker wie beispielsweise Glucamin.

Als **Konservierungsmittel** eignen sich beispielsweise Phenoxyethanol, Formaldehydlösung, Parabene, Pentandiol oder Sorbinsäure. Als **Insekten-Repellentien** kommen N,N-Diethyl-m-touluamid, 1,2-Pentandiol oder Insect repellent 3535 in Frage, als **Selbstbräuner** eignet sich Dihydroxyaceton.

Als **Parfümöle** seien genannt die Extrakte von Blüten (Lavendel, Rosen, Jasmin, Neroli), Stengeln und Blättern (Geranium, Patchouli, Petitgrain), Früchten (Anis, Koriander, Kümmel, Wacholder), Fruchtschalen (Bergamotte, Zitrone, Orangen), Wurzeln (Macis, Angelica, Sellerie, Kardamon, Costus, Iris, Calmus), Hölzern (Sandel-, Guajak-, Zedern-, Rosenholz), Kräutern und Gräsern (Estragon, Lemongras, Salbei, Thymian), Nadeln und Zweigen (Fichte, Tanne, Kiefer, Latschen), Harzen und Balsamen (Galbanum, Elemi, Benzoe, Myrrhe, Olibanum, Opoponax). Weiterhin kommen tierische Rohstoffe in Frage, wie beispielsweise Moschus, Zibet und Castoreum. Als synthetische bzw. halbsynthetische Parfümöle kommen Ambroxan, Eugenol, Isoeugenol, Citronellal, Hydroxycitronellal, Geraniol, Citronellol, Geranylacetat, Citral, Ionon und Methylionon in Betracht.

Als **Farbstoffe** können die für kosmetische Zwecke geeigneten und zugelassenen Substanzen verwendet werden, wie sie beispielsweise in der Publikation **"Kosmetische Färbemittel" der Farbstoffkom-mission der Deutschen Forschungsgemeinschaft, Verlag Chemie, Weinheim, 1984, S.81-106** zusammengestellt sind. Diese Farbstoffe werden üblicherweise in Konzentrationen von 0,001 bis 0,1 Gew.-%, bezogen auf die gesamte Mischung, eingesetzt.

Der Gesamtanteil der Hilfs- und Zusatzstoffe kann 1 bis 50, vorzugsweise 5 bis 40 Gew.-% - bezogen auf die Mittel - betragen. Die Herstellung der Mittel kann durch übliche Kalt- oder Heißprozesse erfolgen; vorzugsweise arbeitet man nach der Phaseninversionstemperatur-Methode.

### Beispiele

**Beispiel 1.** In einem 1,5-l-Dreihalskolben mit Rührer, Tropftrichter und Destillationsaufsatz wurden 828 g (4 Mol) Kokosfettsäure und 1,2 g Hypophosphorsäure (50 Gew.-%ig) vorgelegt und auf 70°C erhitzt. Bei einem verminderten Druck von 30 mbar wurden portionsweise 363 g (2,4 Mol) Triethanolamin zugetropft und dabei die Temperatur bis auf 160°C gesteigert. Nach dem Ende der Zugabe wurde die Reaktionsmischung noch weitere 2 h bei 2 mbar gerührt, bis kein weiteres Reaktionswasser abgeschieden wurde und die Säurezahl einen Wert unterhalb von 5 mg KOH/g erreicht hatte. Anschließend wurden 400 g (0,86 Mol) des hergestellten Esters in einen zweiten Dreihalskolben überführt und bei 50°C in 126 g Propylenglycol gelöst. Anschließend wurden portionsweise 104 g (0,83 Mol) Dimethylsulfat zugetropft und die Mischung 4 h bei 65°C gerührt. Es wurde eine bei 20°C klare niedrigviskose Lösung erhalten, die 80 Gew.-% Esterquat und 20 Gew.-% Propylenglycol enthielt.

**Beispiel 2.** Beispiel 1 wurde wiederholt, der resultierende Ester jedoch in 56 g Propylenglycol gelöst und dann quaterniert. Nach Abschluß der Umsetzung mit Dimethylsulfat wurden 422 g Wasser zugegeben, wobei eine ebenfalls klare, niedrigviskose Lösung mit einem Gehalt von 51 Gew.-% Esterquat erhalten wurde.

**Beispiel 3.** Beispiel 1 wurde wiederholt, die Quaternierung jedoch in Gegenwart von 32 g Ethanol durchgeführt und das Reaktionsprodukt anschließend in 515 g Wasser gelöst. Es wurde eine klare, niedrigviskose Lösung erhalten, die 48 Gew.-% Esterquat, 3 Gew.-% Ethanol und 49 Gew.-% Wasser enthielt.

**Beispiel 4.** Beispiel 1 wurde wiederholt, die Quaternierung jedoch in einer Mischung von 71 g Propylenglycol und 18 g Ethanol durchgeführt. Es wurde eine klare, niedrigviskose Lösung erhalten, die 85 Gew.-% Esterquat, 12 Gew.-% Propylenglycol und 3 Gew.-% Ethanol enthielt.

**Beispiel 5.** Analog Beispiel 1 wurden 828 g (4 Mol) Kokosfettsäure und 397 g (2,7 Mol) Triethanolamin umgesetzt. 400 g (0,92 Mol) des resultierenden Esters wurden bei 50°C in 30 g Ethanol gelöst. Anschließend wurden portionsweise 110 g (0,87 Mol) Diemthylsulfat zugegeben und die Mischung 4 h bei 65°C gerührt. Nach Abschluß der Quaternierung wurden portionsweise 315 g Wasser zugegeben, wobei eine niedrigviskose, bei 20°C klare Lösung mit einem Gehalt an Esterquat von 60 Gew.-% erhalten wurde.

**Beispiel 6.** Beispiel 5 wurde wiederholt, anstelle des Ethanols jedoch 55 g Propylenglycol eingesetzt. Nach Abschluß der Quaternierung wurden der Mischung 220 g Wasser zugegeben, wobei eine niedrigviskose, klare Lösung mit 65 Gew.-% Esterquat, 7 Gew.-% Propylenglycol und 28 Gew.-% Wasser erhalten wurde.

**Beispiel 7.** Analog Beispiel 1 wurden 900 g (4,5 Mol) Laurinsäure, 100g (0,5 Mol) Kokosfettsäure und 453 g (3 Mol) Triethanolamin zur Reaktion gebracht. 400 g (0,88 Mol) des resultierenden Esters wurden bei 50°C in 57 g Propylenglycol gelöst und protionsweise mit 105 g (0,83 Mol) Diemthylsulfat versetzt. Nach Abschluß der Quaternierung wurden der Mischung 700 g Wasser zugesetzt, wobei eine klare, niedrigviskose Lösung erhalten wurde, die 40 Gew.-% Esterquat, 4,5 Gew.-% Propylenglycol und 55,5 Gew.-% Wasser enthielt.

## Patentansprüche

1. Wassermischbare Kationtensidkonzentrate, enthaltend
(a) 75 bis 95 Gew.-% Esterquats und
(b) 5 bis 25 Gew.-% Ethanol, Ethylenglycol und/oder Propylenglycol
mit der Maßgabe, daß sich die Mengenangaben zu 100 Gew.-% ergänzen.

2. Konzentrate nach Anspruch 1, **dadurch gekennzeichnet,** daß sie Esterquats der Formel **(I)** enthalten, 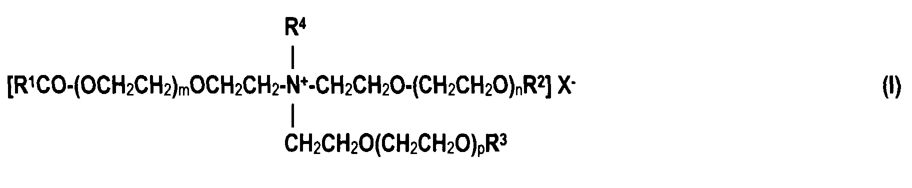 in der R¹CO für einen Acylrest mit 6 bis 22 Kohlenstoffatomen, R² und R³ unabhängig voneinander für Wasserstoff oder R¹CO, R⁴ für einen Alkylrest mit 1 bis 4 Kohlenstoffatomen oder eine (CH₂CH₂O)_{q}H-Gruppe, m, n und p in Summe für 0 oder Zahlen von 1 bis 12, q für Zahlen von 1 bis 12 und X für Halogenid, Alkylsulfat oder Alkylphosphat steht.

3. Konzentrate nach Anspruch 1**, dadurch** gekennzeichnet, daß sie Esterquats der Formel **(II)** enthalten, 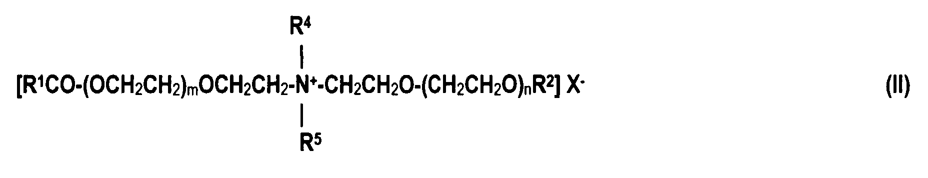 in der R¹CO für einen Acylrest mit 6 bis 22 Kohlenstoffatomen, R² für Wasserstoff oder R¹CO, R⁴ und R⁵ unabhängig voneinander für Alkylreste mit 1 bis 4 Kohlenstoffatomen, m und n in Summe für 0 oder Zahlen von 1 bis 12 und X für Halogenid, Alkylsulfat oder Alkylphosphat steht.

4. Konzentrate nach Anspruch 1, **dadurch gekennzeichnet,** daß sie Esterquats der Formel **(III)** enthalten, 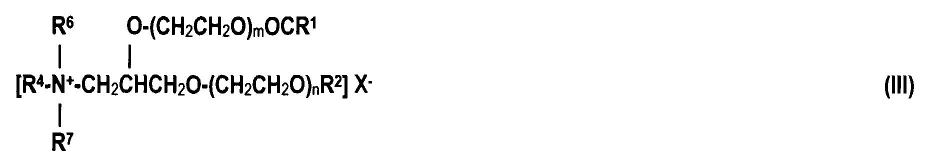 in der R¹CO für einen Acylrest mit 6 bis 22 Kohlenstoffatomen, R² für Wasserstoff oder R¹CO, R⁴, R⁶ und R⁷ unabhängig voneinander für Alkylreste mit 1 bis 4 Kohlenstoffatomen, m und n in Summe für 0 oder Zahlen von 1 bis 12 und X für Halogenid, Alkylsulfat oder Alkylphosphat steht.

5. Verfahren zur Herstellung von Konzentraten nach Anspruch 1, **dadurch gekennzeichnet,** daß man Alkanolaminfettsäureester in Gegenwart von solchen Mengen Ethanol, Ethylenglycol, Propylenglycol oder deren Gemischen mit Alkylierungsmitteln umsetzt, daß sich ein Gewichtsverhältnis Esterquat : Lösemittel von 95 : 5 bis 75 : 25 ergibt.

6. Kosmetische Zubereitungen, enthaltend
(a) 40 bis 75 Gew.-% Konzentrate nach Anspruch 1 und
(b) 25 bis 60 Gew.-% Wasser,
mit der Maßgabe, daß sich die Mengenangaben zu 100 Gew.-% ergänzen.

7. Verwendung der Konzentrate nach den Ansprüchen 1 bis 4 zur Herstellung von kosmetischen Zubereitungen.
